# EUROPEAN PATENT APPLICATION

(11) **EP 4 292 541 A1**
(43) Date of publication of application: **20.12.2023**
(21) Application number: 22752526.8
(22) Date of filing: 17.01.2022
(51) Int. Cl.: A61B 17/00, A61B 17/94

(54) **ULTRASONIC TREATMENT TOOL DEVICE AND METHOD FOR DRIVING SAME**

(30) Priority: 10.02.2021 JP 2021019838
(71) Applicant: FUJIFILM Corporation, Tokyo 106-8620 (JP)
(72) Inventor: HIGASHI, Kohei, Ashigarakami-gun, Kanagawa 258-8577 (JP); NAKAMURA, Koichiro, Ashigarakami-gun, Kanagawa 258-8577 (JP)
(74) Representative: Meissner Bolte Partnerschaft mbB
(86) International application number: PCT/JP2022/001344
(87) International publication number: WO 2022/172693

(57) **Abstract**

Provided are an ultrasonic treatment tool device capable of raising a temperature of a structure in a short time using an existing device or equipment, and a method for driving the same.

Ultrasound oscillators (51a, 51b) emit ultrasonic waves. An ultrasonic driving part (19) drives the ultrasound oscillators (51a, 51b) such that the ultrasonic waves propagates in a first direction D1 from one side toward the other side and propagates in a second direction D2 opposite to the first direction D1 in a structure S. The ultrasonic driving part (19) drives the ultrasound oscillators (51a, 51b) by changing frequencies of the ultrasound oscillators (51a, 51b) such that a current value flowing through the ultrasound oscillators (51a, 51b) is a maximal current value.

## Description

### BACKGROUND OF THE INVENTION

### 1. Field of the Invention

The present invention relates to an ultrasonic treatment tool device that raises a temperature of a structure such as a blood vessel to seal the structure, and a method for driving the same.

### 2. Description of the Related Art

In a medical field, it is known that various treatments are performed on a subject by using an ultrasonic treatment tool that generates ultrasonic vibration. WO2019/055870A discloses an ultrasonic treatment tool comprising a pair of grip parts for gripping a structure such as a blood vessel and an ultrasound oscillator (ultrasonic transducer), and configured to transmit ultrasonic vibration through the grip parts and to perform a treatment such as sealing or incision of the blood vessel or the like. The ultrasound oscillator is composed of various devices such as a piezoelectric material, and converts supplied power into ultrasonic vibration.

### SUMMARY OF THE INVENTION

In the ultrasonic treatment tool as disclosed in WO2019/055870A, there is a need to raise a temperature of the grip part in a short time to about 60°C to 90°C, which is a denaturation temperature of a protein or the like, in order to seal the structure such as the blood vessel gripped by the grip parts. In order to raise the temperature of the grip part in a short time, each device in the ultrasound oscillator needs to be supplied with as much power as possible. Therefore, it has been required to raise the temperature of the structure in a short time by supplying a large amount of power to the ultrasound oscillator using an existing device or equipment without separately using a device for supplying a large amount of power.

An object of the present invention is to provide an ultrasonic treatment tool device capable of raising a temperature of a structure in a short time using an existing device or equipment without separately using a device for supplying a large amount of power in a case in which the temperature of the structure is raised by propagating ultrasonic waves to the structure, and a method for driving the same.

An ultrasonic treatment tool device according to an aspect of the present invention comprises: a grip part that grips a structure in a subject by a pair of gripping pieces provided at a distal end portion of the grip part; an ultrasound oscillator that is provided in at least one of a pair of the grip parts and emits an ultrasonic wave; and a processor that drives the ultrasound oscillator such that the ultrasonic wave propagates in a first direction from one side toward the other side and propagates in a second direction opposite to the first direction in the structure, and that performs a frequency fluctuation mode for driving the ultrasound oscillator by changing a frequency of the ultrasound oscillator such that a current value flowing through the ultrasound oscillator is a maximal current value.

It is preferable that in the frequency fluctuation mode, the frequency of the ultrasound oscillator is changed in a frequency range including a resonance frequency of the ultrasound oscillator. It is preferable that the processor monitors the current value of an overlapping portion between a first range showing a waveform of the current value for the ultrasonic wave in the first direction and a second range showing a waveform of the current value for the ultrasonic wave in the second direction, and performs a control to maintain the frequency of the ultrasound oscillator at which the current value of the overlapping portion is the maximal current value. It is preferable that the structure includes a blood vessel. It is preferable that the ultrasonic treatment tool device is an endoscope treatment tool that is inserted into the subject through a forceps channel of an endoscope.

A method for driving an ultrasonic treatment tool device according to an aspect of the present invention is a method for driving an ultrasonic treatment tool device including a grip part that grips a structure in a subject by a pair of gripping pieces provided at a distal end portion of the grip part, and an ultrasound oscillator that is provided in at least one of a pair of the grip parts and emits an ultrasonic wave, the method comprising: via a processor that drives the ultrasound oscillator such that the ultrasonic wave propagates in a first direction from one side toward the other side and propagates in a second direction opposite to the first direction in the structure, a step of performing a frequency fluctuation mode for driving the ultrasound oscillator by changing a frequency of the ultrasound oscillator such that a current value flowing through the ultrasound oscillator is a maximal current value.

According to the present invention, it is possible to raise a temperature of a structure in a short time using an existing device or equipment without separately using a device for supplying a large amount of power in a case in which the temperature of the structure is raised by propagating ultrasonic waves to the structure.

### BRIEF DESCRIPTION OF THE DRAWINGS

Fig. 1 is an explanatory diagram showing a configuration of an endoscope system.
Fig. 2 is a front view of an endoscope and an ultrasonic treatment tool according to the present invention.
Figs. 3A and 3B are cross-sectional views of a main part of the ultrasonic treatment tool, where Fig. 3A is a cross-sectional view of a main part showing a state in which a grip part is closed, and Fig. 3B is a cross-sectional view of a main part showing a state in which the grip part is opened.
Fig. 4 is a perspective view of the ultrasonic treatment tool in the state in which the grip part is closed.
Fig. 5 is a perspective view of the ultrasonic treatment tool in the state in which the grip part is opened.
Fig. 6 is a cross-sectional view taken along the line VI-VI of Fig. 3A.
Fig. 7 is a block diagram showing an outline of an ultrasonic driving part.
Fig. 8 is a flowchart showing a flow of a frequency fluctuation mode.
Fig. 9 is a graph showing a waveform of a current value in a case in which an ultrasonic wave is emitted from an ultrasound oscillator.
Fig. 10 is a cross-sectional view of an ultrasonic treatment tool for gripping a structure.

### DESCRIPTION OF THE PREFERRED EMBODIMENTS

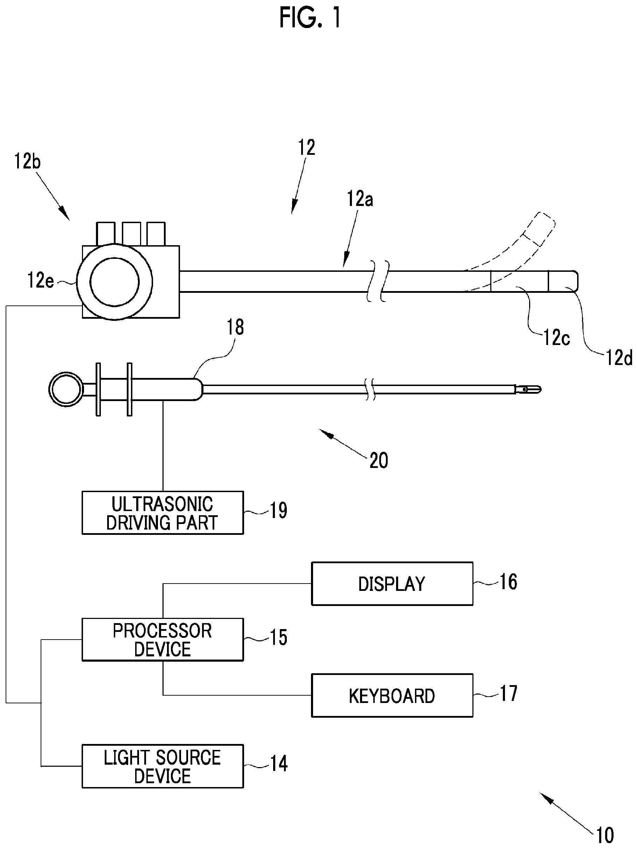

As shown in Fig. 1, an endoscope system 10 comprises an endoscope 12, a light source device 14, a processor device 15, a display 16, a user interface 17, and an ultrasonic treatment tool device 20. The endoscope 12 images an observation target. The light source device 14 emits illumination light with which the observation target is irradiated. The processor device 15 performs a system control of the endoscope system 10. The display 16 is a display unit that displays an observation image or the like based on an endoscopic image. The user interface 17 is a mouse, a touch pad, a keyboard, or the like, and is an input device that performs setting input to the processor device 15 or the like.

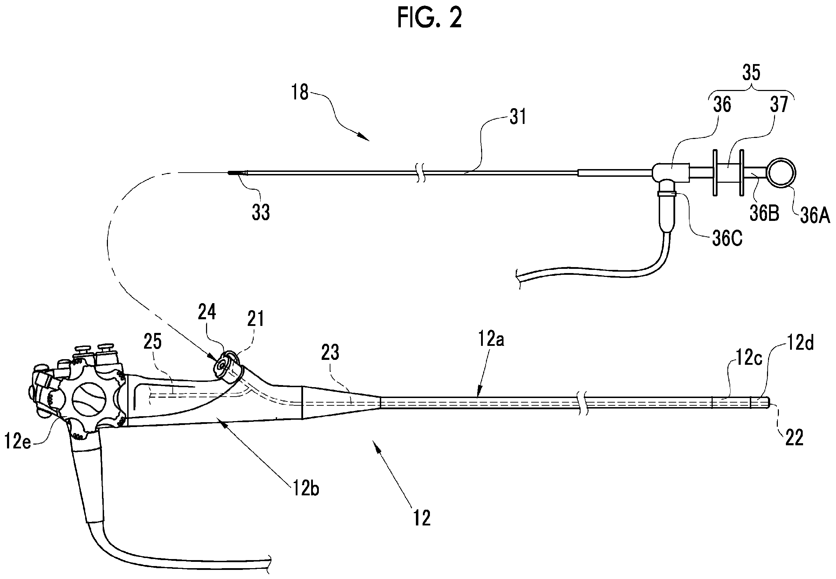

The endoscope 12 is optically connected to the light source device 14 and electrically connected to the processor device 15. The endoscope 12 includes an insertion part 12a to be inserted into a subject, an endoscope operating part 12b provided at a base end portion of the insertion part 12a, a bendable part 12c provided on a distal end side of the insertion part 12a, and a distal end portion 12d. By operating an angle knob 12e of the endoscope operating part 12b, the bendable part 12c is bent. As a result, the distal end portion 12d is directed in a desired direction. In addition to the angle knob 12e, a forceps port 21 (see Fig. 2) is provided in the endoscope operating part 12b. The forceps port 21 is an entrance into which the ultrasonic treatment tool 18 is inserted. The ultrasonic treatment tool 18 inserted into the forceps port 21 protrudes from a forceps outlet 22 (see Fig. 2) of the distal end portion 12d.

An observation window and an illumination window are provided on a distal end surface of the distal end portion 12d, although not shown. An image sensor (not shown) or the like is disposed on the inside of the observation window, and an optical fiber cable (not shown) is disposed on the inside of the illumination window. A signal line of the image sensor and the optical fiber cable are connected to the processor device 15 and the light source device 14, respectively.

The processor device 15 is electrically connected to the display 16 and the user interface 17. The processor device 15 performs image processing or the like on an endoscopic image captured by the image sensor and displays the processed image on the display 16. The ultrasonic treatment tool device 20 comprises an ultrasonic treatment tool 18 which is one endoscope treatment tool to be inserted into the subject through a forceps channel 23 (see Fig. 2) of the endoscope, and an ultrasonic driving part 19 that supplies power to the ultrasonic treatment tool 18.

As shown in Fig. 2, a forceps channel 23 for inserting the ultrasonic treatment tool 18 is disposed in the insertion part 12a. One end of the forceps channel 23 is connected to the forceps outlet 22, and the other end of the forceps channel 23 is connected to the forceps port 21 provided in the endoscope operating part 12b. The forceps port 21 is provided with a forceps valve 24. The forceps channel 23 is also used as a route for feeding a cleaning liquid such as water from the forceps outlet 22 and a route for sucking a body fluid such as blood and contents such as body waste materials. The ultrasonic driving part 19 (see Fig. 1) supplies power to an ultrasound oscillator 51, which will be described below, of the ultrasonic treatment tool 18.

The ultrasonic treatment tool 18 is an endoscope treatment tool to be inserted into a subject together with the insertion part 12a through the forceps channel 23. In the present embodiment, the ultrasonic treatment tool 18 is mentioned as the endoscope treatment tool to be combined with the endoscope 12, but, in practice, the present invention is not limited to this, and treatment tools such as biopsy forceps, snares, or electric scalpels are also combined with the endoscope 12.

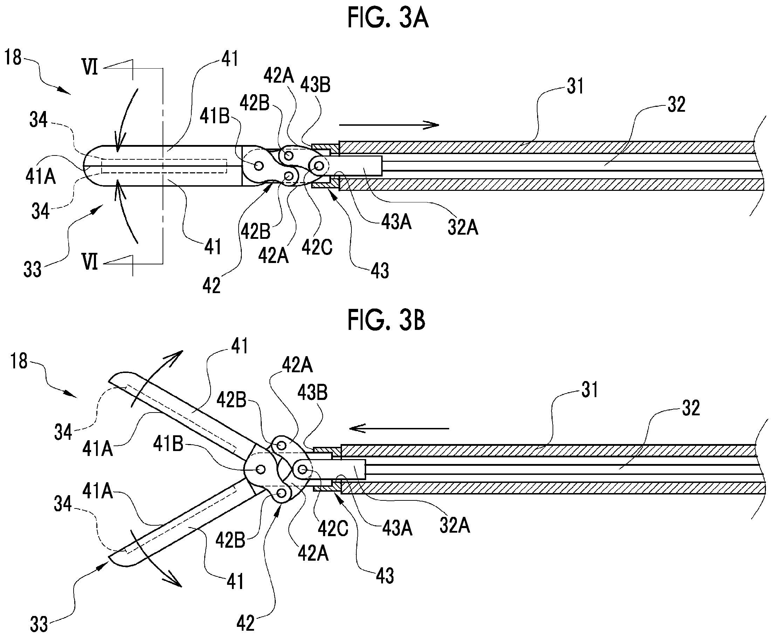

The ultrasonic treatment tool 18 comprises a flexible sheath 31, an operation wire 32 (see Figs. 3A and 3B), a grip part 33, an ultrasound oscillator unit 34 (see Figs. 3A and 3B), and a treatment tool operating part 35. The flexible sheath 31 is a tubular sheath formed of a flexible material, for example, a soft resin, and is inserted into the forceps channel 23 of the endoscope 12. The operation wire 32 is provided integrally with the grip part 33 and is inserted through the flexible sheath 31.

The treatment tool operating part 35 comprises an operating part main body 36 and a slider 37 that is slidably supported by the operating part main body 36. The operating part main body 36 is installed consecutively to a base end portion of the flexible sheath 31. The operating part main body 36 is provided with a finger hook portion 36A, a cylindrical portion 36B, and a connector portion 36C. The cylindrical portion 36B extends in a direction parallel to an axial direction of the flexible sheath 31. The slider 37 is engaged with the cylindrical portion 36B and slidably moves along the cylindrical portion 36B in the axial direction of the flexible sheath 31. In a case in which a patient is treated, a thumb of a user is hooked on the finger hook portion 36A, and an index finger and a middle finger of the same user are hooked on the slider 37. A base end of the operation wire 32 is fixed to the slider 37. Therefore, the operation wire 32 is pushed and pulled in the flexible sheath 31 in the axial direction with the sliding movement of the slider 37. The connector portion 36C is electrically connected to the ultrasonic driving part 19 (see Fig. 1).

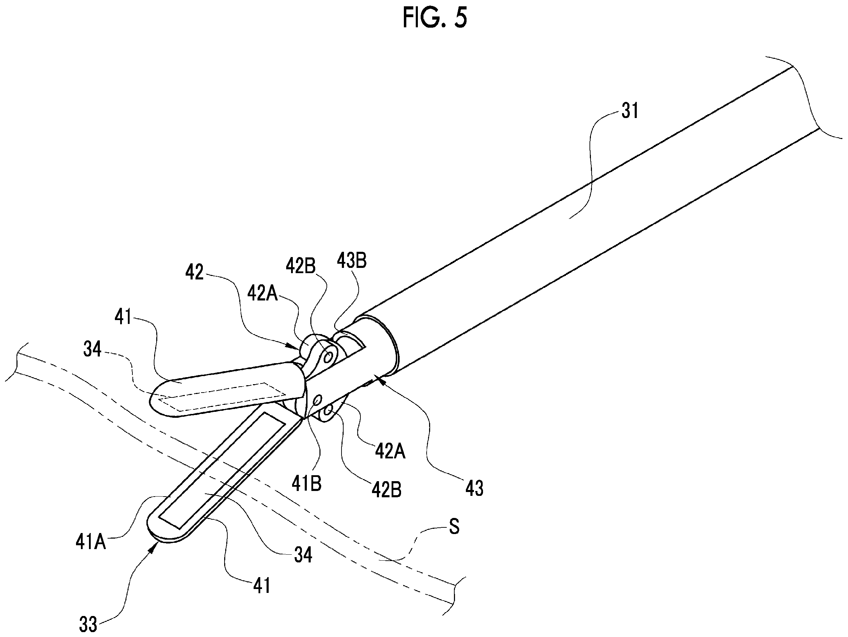

As shown in Figs. 3A and 3B, the grip part 33 is provided at a distal end portion of the flexible sheath 31, and comprises a pair of gripping pieces 41 disposed up and down, a link mechanism 42, and a support member 43 that supports the gripping pieces 41 and the link mechanism 42. The ultrasound oscillator unit 34 is provided on inner surfaces 41A of the pair of gripping pieces 41, that is, surfaces facing each other. In a case in which the grip part 33 is closed, the inner surfaces 41A of the pair of gripping pieces 41 abut on each other. Thereby, a structure S (see Fig. 5) such as a blood vessel can be gripped by being interposed between the pair of gripping pieces 41. The pair of gripping pieces 41 constituting the grip part 33 can be opened and closed in a vertical direction about a support shaft 41B. The support shaft 41B is supported by the support member 43. In a case in which the grip part 33 is closed, each of the gripping pieces 41 is formed in a semi-cylindrical shape such that an outer peripheral surface thereof has a continuous cylindrical shape.

The link mechanism 42 comprises a link plate 42A, a connecting pin 42B, and a fitting pin 42C. One end portion of the link plate 42A is connected to the pair of gripping pieces 41 via the connecting pin 42B. A position at which the pair of gripping pieces 41 is connected to the link plate 42A is a base end portion of the gripping pieces 41, which is located on a base end side with respect to the support shaft 41B. The other end portion of the link plate 42A is connected to a connecting member 32A provided at a distal end of the operation wire 32 via the fitting pin 42C. The fitting pin 42C rotatably connects the link plate 42A to the connecting member 32A.

The connecting member 32A is formed in a cylindrical shape. The connecting member 32A is partially located inside the flexible sheath 31 through a through-hole 43A formed in the support member 43. The support member 43 is formed in a substantially cylindrical shape and is fixed to a distal end of the flexible sheath 31. The support member 43 has a notch 43B notched from a distal end thereof. Since the pair of gripping pieces 41 and the link plate 42A move inside the notch 43B, the support member 43 does not hinder the movement of the gripping pieces 41 and the link plate 42A.

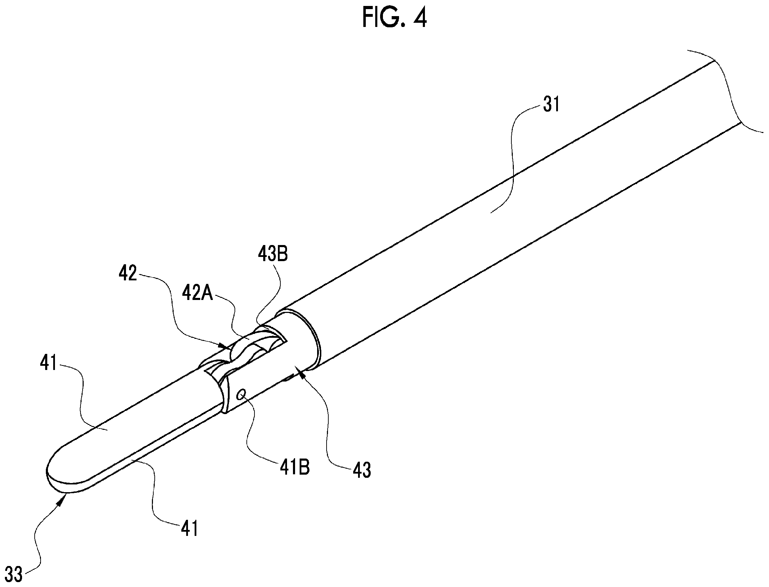

The link mechanism 42 converts a linear motion caused by the pushing-pulling operation of the operation wire 32 into a rotational motion, and opens and closes the gripping pieces 41. That is, in a case in which the slider 37 is pulled toward the finger hook portion 36A, the gripping pieces 41 are closed to close the grip part 33 as shown in Figs. 3A and 4. On the contrary, in a case in which the slider 37 is pushed toward the grip part 33, the gripping pieces 41 are opened to open the grip part 33 as shown in Figs. 3B and 5.

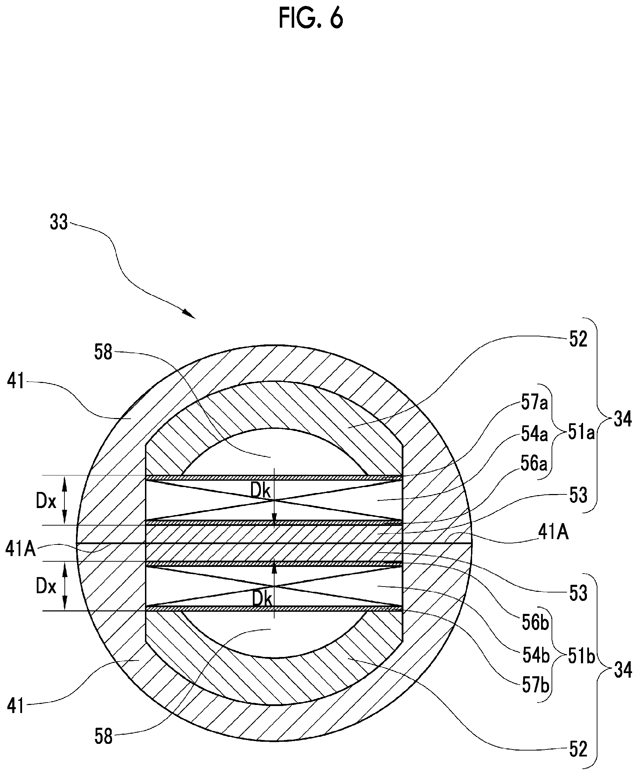

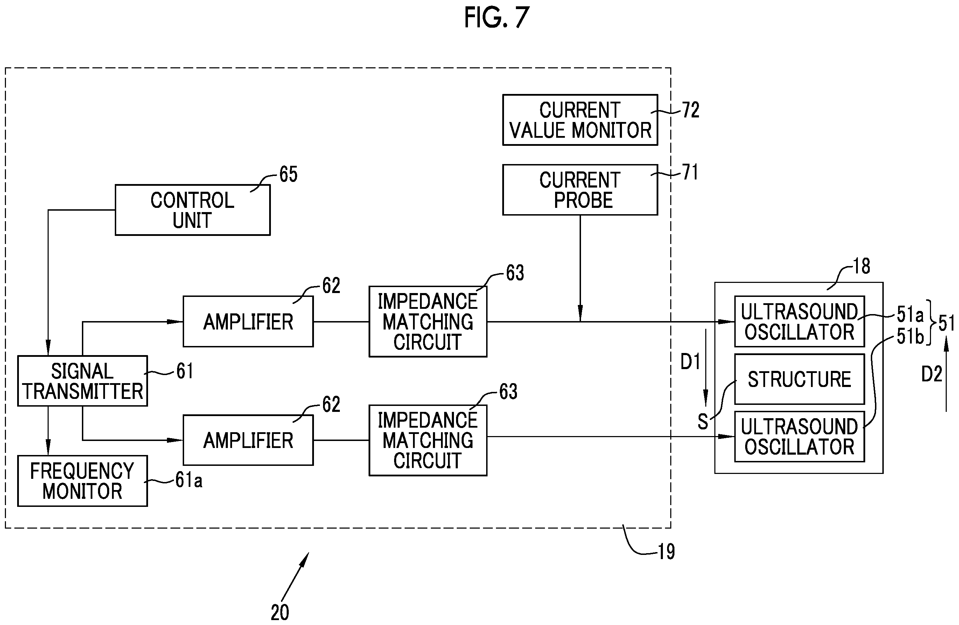

As shown in Fig. 6, the ultrasound oscillator unit 34 is configured by laminating the ultrasound oscillator 51 (see Fig. 7), a backing material layer 52, and an acoustic matching layer 53.

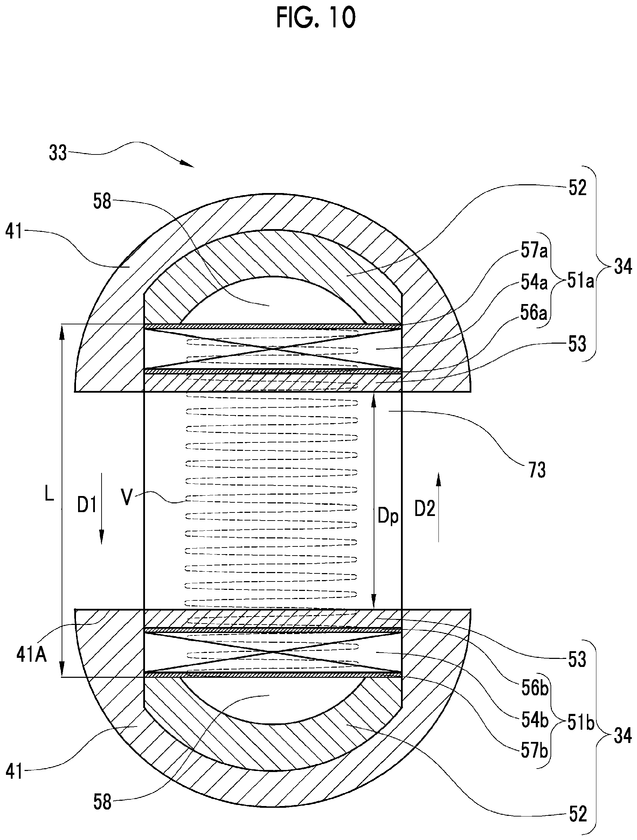

The ultrasound oscillator 51 is composed of two ultrasound oscillators 51a and 51b facing each other. The ultrasound oscillator 51a is composed of a piezoelectric material 54a (also referred to as a piezoelectric element) and electrodes 56a and 57a. The piezoelectric material 54a is formed in a plate shape. The electrodes 56a and 57a are formed in a plate shape thinner than the piezoelectric material 54a, and are laminated on both surfaces of the piezoelectric material 54a. The ultrasound oscillator 51a is disposed in a parallel to the inner surface 41A of the gripping piece 41 so as to face a structure such as a blood vessel gripped by the grip part 33. In addition, a direction Dk in which the ultrasound oscillator 51 vibrates is parallel to a direction Dx in which the piezoelectric material 54a and the electrodes 56a and 57a are laminated. Then, in a case in which the structure S is gripped by the grip part 33, the ultrasonic waves emitted from the ultrasound oscillator 51a vibrate in a first direction D1 (direction from the ultrasound oscillator 51a to the ultrasound oscillator 51b) along a cross-sectional direction of the structure S, or the ultrasonic waves emitted from the ultrasound oscillator 51b vibrate in a second direction D2 (direction from the ultrasound oscillator 51b toward the ultrasound oscillator 51a) (see Fig. 10). For example, in a case in which the structure S is a biomimetic material 73, a cross-sectional direction Dp of the biomimetic material 73 is the cross-sectional direction of the structure S (see Fig. 10).

The electrodes 56a and 57a are connected to a connector portion 36C (see Fig. 2) of the treatment tool operating part 35 via a signal cable (not shown). The signal cable is wired along an inner peripheral surface or an outer peripheral surface of the flexible sheath 31, for example. In a case in which the ultrasonic driving part 19 is connected to the connector portion 36C, the electrodes 56a and 57a are electrically connected to the ultrasonic driving part 19 via the signal cable and the connector portion 36C. Of the electrodes 56a and 57a, one is connected to the ground via the signal cable or the like, and the other is supplied with power of an AC voltage signal, which is described below, from the ultrasonic driving part 19. The ultrasound oscillator 51b is composed of a piezoelectric material 54b and electrodes 56b and 57b. The piezoelectric material 54b is the same as the piezoelectric material 54a, and the electrodes 56b and 57b are the same as the electrodes 56a and 57a.

The acoustic matching layer 53 is provided to achieve acoustic impedance matching between a human body of a patient and the ultrasound oscillator 51. The acoustic matching layer 53 is disposed outside the ultrasound oscillator 51, and strictly speaking, the acoustic matching layer 53 is superposed on a side facing a structure gripped by the grip part 33 with respect to the ultrasound oscillator 51. That is, the acoustic matching layer 53 is provided at a position exposed from the inner surface 41A of the gripping piece 41.

Since the acoustic matching layer 53 is provided, it is possible to increase a transmittance of the ultrasonic wave. As a material of the acoustic matching layer 53, various organic materials whose acoustic impedance values are closer to that of the human body of the patient than the piezoelectric material of the ultrasound oscillator 48 can be used. Specific examples of the material of the acoustic matching layer 53 include epoxy resin, silicone rubber, polyimide, and polyethylene. In addition, the acoustic matching layer 53 is formed of a plurality of layers, and the material and the number of formed layers are appropriately selected according to a required acoustic impedance value.

The backing material layer 52 supports the ultrasound oscillator 51 from a back side (a side opposite to the acoustic matching layer 53). A backing material is made of, for example, a rigid material such as hard rubber. In addition, an air gap layer 58, that is, a gap interposed between the backing material layer 52 and the ultrasound oscillator 51 is formed between the backing material layer 52 and the ultrasound oscillator 51. Since the air gap layer 58 can reflect the ultrasonic wave by internal air, it has a function of reflecting the ultrasonic wave emitted from the back side of the ultrasound oscillator 51. Accordingly, the ultrasonic vibration can be efficiently transmitted to a structure such as a blood vessel S. Note that the present invention is not limited to this, and the backing material layer 52 may be filled with a material for reflecting the ultrasonic wave without providing the air gap layer 58.

As shown in Fig. 7, in the ultrasonic treatment tool device 20, the ultrasonic treatment tool 18 having the ultrasound oscillator 51 is electrically connected to the ultrasonic driving part 19. Accordingly, power is supplied from the ultrasonic driving part 19 to the ultrasonic treatment tool 18.

The ultrasonic driving part 19 comprises a signal transmitter 61, an amplifier 62, an impedance matching circuit 63, and a control unit 65. The signal transmitter 61 has a function of generating an AC voltage signal having an optional frequency and waveform, and has, for example, the same configuration and function as a known function generator. A frequency actually used while driving the ultrasound oscillator 51 is displayed on a frequency monitor 61a. It is preferable that the ultrasonic driving part 19 is provided with a current probe 71 and a current value monitor 72 composed of an oscilloscope or the like.

The ultrasonic driving part 19 stores programs related to various kinds of processing in a program memory (not shown). The control unit 65 configured by the processor executes the program in the program memory to realize the functions of the signal transmitter 61, the amplifier 62, and the impedance matching circuit 63.

The signal transmitter 61 outputs, for example, an AC voltage signal with a waveform of a sin wave. In order to supply power to a pair of the ultrasound oscillators 51a and 51b, the amplifier 62 and the impedance matching circuit 63 are provided for each of the ultrasound oscillators 51a and 51b. The signal transmitter 61 outputs AC voltage signals having the same frequency and the same waveform to the amplifiers 62, respectively. The amplifier 62 amplifies the AC voltage signal output from the signal transmitter 61 to a voltage at a level at which the ultrasound oscillators 51a and 51b can be driven. The impedance matching circuit 63 is connected in series with the amplifier 62, and can match an input impedance of the AC voltage signal output from the amplifier 62 with an impedance of the ultrasound oscillators 51a and 51b.

In a case in which a frequency fluctuation mode is set as an operation mode that can be set, the control unit 65 is provided with a frequency fluctuation mode for driving the ultrasound oscillator 51 by changing the frequency of the ultrasound oscillator 51 such that a current value flowing through the ultrasound oscillator 51a is a maximal current value in a fixed frequency range with respect to a frequency of the ultrasound oscillator 51a. It is preferable that the detection of the maximal current value in executing the frequency fluctuation mode is performed within a fixed time from a start of setting of the frequency fluctuation mode. The largest current value among the current values detected within a fixed time is defined as the maximal current value. In the middle of the treatment using the ultrasonic treatment tool 18, it is also assumed that a state of a blood vessel or the ultrasound oscillator 51 changes (for example, an influence of heating) and the optimum driving conditions of the ultrasonic treatment tool 18 change. Therefore, the frequency fluctuation mode is provided to cope with such a situation. It is preferable that the control unit 65 executes the frequency fluctuation mode in accordance with a control program for the frequency fluctuation mode.

In frequency scanning in which the frequency of the ultrasound oscillator 51 is changed, it is preferable that a resonance frequency of the ultrasound oscillator 51 is set as a center frequency and the frequency is changed within a frequency range including the center frequency. The frequency range is determined between a frequency lower limit value smaller than the center frequency and a frequency upper limit value larger than the center frequency. The current value flowing through the ultrasound oscillator 51a is preferably a current value flowing through the ultrasound oscillator 51a in a section between the impedance matching circuit 63 and the ultrasound oscillator 51a, and the current value in the section is preferably monitored by the current probe 71. In a case in which the current value flowing through the ultrasound oscillator 51a is equal to or close to the maximal current value, it is preferable to stop the frequency scanning at that timing to fix the frequency. On the other hand, in a case in which the current value flowing through the ultrasound oscillator 51a changes again from the maximal current value, it is preferable to restart the frequency scanning. A calculation method of the resonance frequency will be described below.

The ultrasonic waves emitted from the ultrasound oscillator 51a propagate in the first direction D1 from one side toward the other side in the structure S. On the other hand, the ultrasonic waves emitted from the ultrasound oscillator 51b propagate in the second direction D2 opposite to the first direction. In the current value monitor 72, the time is represented by a horizontal axis and the current value is represented by a vertical axis, and the current value at a predetermined frequency is displayed. The current value increases or decreases at fixed intervals with respect to the frequency scanning. It is considered that this is because the ultrasonic waves are strengthened or weakened by a relationship between a wavelength of the ultrasonic waves emitted from the ultrasound oscillators 51a and 51b and a distance at which the ultrasonic waves propagate. It is considered that the impedance of the piezoelectric material 54 decreases at a frequency at which the ultrasonic waves are strengthened, and as a result, the current value increases. In a case in which the propagation direction of the ultrasonic waves is only one of the first direction D1 or the second direction D2, no change in the current value occurs.

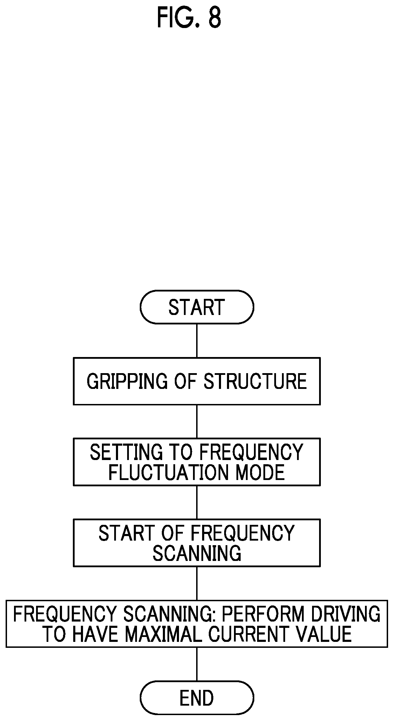

Hereinafter, a flow of a driving method in a case of actually using the ultrasonic treatment tool 18 will be described with reference to a flowchart of Fig. 8. First, the structure S is gripped by the grip part 33 by the ultrasonic treatment tool 18. In a case of gripping the structure S, the ultrasound oscillators 51a and 51b facing each other are configured to have a specific positional relationship. In a case in which the structure S has a certain thickness, the specific positional relationship preferably includes a positional relationship of the ultrasound oscillators 51a and 51b opened at a specific angle (for example, more than 0 degrees but less than 90 degrees) about the support shaft 41B in accordance with the thickness of the structure S. In a case of the ultrasonic treatment tool 18 of a type in which the support shaft 41B is not used for the opening and closing operation of the ultrasound oscillators 51a and 51b, the specific positional relationship includes a positional relationship in which the ultrasound oscillators 51a and 51b are parallel to each other.

Next, the control unit 65 is set to the frequency fluctuation mode, and frequency scanning is performed in which the ultrasound oscillators 51a and 51b are driven by changing the frequencies. In the frequency scanning, the current probe 71 is mounted to a probe mounting portion of the ultrasonic treatment tool 18 so that the current value flowing between the impedance matching circuit 63 and the ultrasound oscillator 51a can be measured. In addition, the current value detected by the current probe 71 is displayed on the current value monitor 72.

Subsequently, in the frequency scanning, the drive control is performed such that the current value flowing through the ultrasound oscillators 51a and 51b is the maximal current value. That is, the control unit 65 performs a control of driving the ultrasound oscillator 51 with a constant voltage and driving the ultrasound oscillators 51a and 51b by changing the frequencies of the ultrasound oscillators 51a and 51b such that a current value flowing through the ultrasound oscillators 51a and 51b is the maximal current value. The frequency range in which the frequencies of the ultrasound oscillators 51a and 51b are changed is a range including the resonance frequencies of the ultrasound oscillators 51a and 51b. Then, in a case in which the maximal current value is reached, the frequency scanning is stopped to fix the frequencies of the ultrasound oscillators 51a and 51b. Accordingly, a temperature of the structure S gripped by the grip part 33 can be raised in a short time.

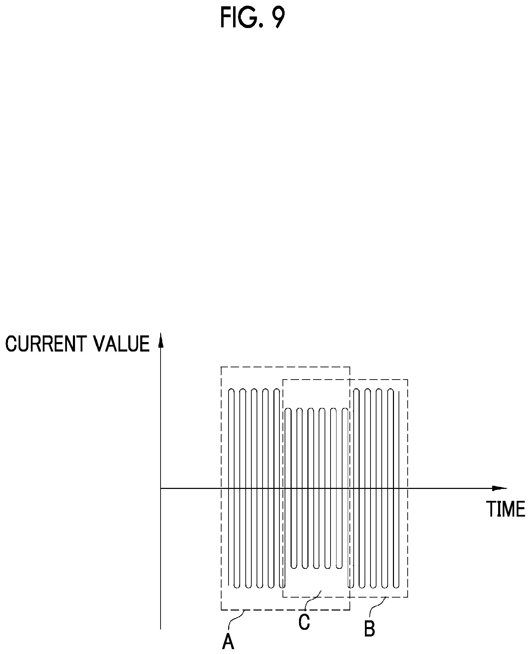

Hereinafter, a relationship between the ultrasonic wave propagating to the structure S and the current value will be described in detail. In Fig. 9, a vertical axis represents the current value flowing through the ultrasound oscillators 51a and 51b, and a horizontal axis represents the time during which the ultrasonic waves are transmitted from the ultrasound oscillators 51a and 51b. The time corresponding to a first range A is the time during which the ultrasonic wave in the first direction D1 (see Fig. 7) is transmitted. Therefore, the first range A represents the waveform of the current value of the ultrasonic wave propagating in the first direction D1. In addition, the time corresponding to a second range B is the time during which the ultrasonic wave in the second direction D2 (see Fig. 7) is transmitted. Therefore, the second range B represents the waveform of the current value of the ultrasonic wave in which the ultrasonic wave in the second direction D2 propagates. The time corresponding to an overlapping portion C between the first range A and the second range B is the time during which both the ultrasonic wave in the first direction D1 and the ultrasonic wave in the second direction D2 are transmitted. Therefore, the overlapping portion C represents the waveform of the current value of the ultrasonic wave in which the ultrasonic wave in the first direction D1 and the ultrasonic wave in the second direction D2 are combined.

The control unit 65 monitors the current value of the overlapping portion C, and performs a control to maintain the frequency of the ultrasound oscillator 51 at which the current value of the overlapping portion is the maximal current value. In this case, in order to detect the maximal current value, a monitoring period is preferably within a fixed time from a start of setting the frequency fluctuation mode.

Hereinafter, a method of calculating the resonance frequency will be described. As shown in Fig. 10, assuming that a distance between the electrodes 57a and 57b is L, a frequency of the ultrasound oscillator 51 is f, a sound speed of an ultrasonic wave V is c, and a phase shift of a terminal reflecting portion is θ in a case in which the biomimetic material 73 simulating the structure S on a living body is gripped by the grip part 33, in a case in which the ultrasonic wave is emitted from both the ultrasound oscillators 51a and 51b, a condition (resonance condition) for the resonance of the ultrasonic wave in the first direction D1 and the ultrasonic wave in the second direction D2 is that "(L × f/c) + θ" is an integer (N) (N = (L × f/c) + θ). Therefore, in a case in which the ultrasonic wave is emitted from both the ultrasound oscillators 51a and 51b, a resonance frequency fr, which is the frequency of the ultrasound oscillator 51 in a case in which the resonance condition is satisfied, can be expressed as "(c × (N - θ))/L". The phase shift θ of the terminal reflecting portion in a case in which the terminal reflecting portion is a fixed end is set to "0", and the phase shift θ of the terminal reflecting portion in a case in which the terminal reflecting portion is a free end is set to "0.5". The terminal reflecting portion corresponds to the electrodes 57a and 57b.

In a case in which the ultrasonic wave is emitted only from any one of the ultrasound oscillator 51a or the ultrasound oscillator 51, the resonance condition is that "(2L × f/c) + θ" is an integer (N) (N = (2L × f/c) + θ). Therefore, in a case in which the ultrasonic wave is emitted only from any one of the ultrasound oscillator 51a or the ultrasound oscillator 51, the resonance frequency fr in a case in which the resonance condition is satisfied can be expressed as "(c × (N - θ))/2L".

In the above-described embodiment, a hardware structure of a processing unit that executes various kinds of processing, such as the signal transmitter 61, the amplifier 62, the impedance matching circuit 63, and the control unit 65, is various processors as shown in below. The various processors include a central processing unit (CPU) that is a general-purpose processor that executes software (programs) to function as various processing units, a graphical processing unit (GPU), a programmable logic device (PLD) that is a processor capable of changing a circuit configuration after manufacture, such as a field programmable gate array (FPGA), and an exclusive electric circuit that is a processor having a circuit configuration exclusively designed to execute various kinds of processing.

One processing unit may be configured of one of these various processors, or may be configured of a combination of two or more processors of the same type or different types (for example, a plurality of FPGAs, a combination of a CPU and an FPGA, or a combination of a CPU and a GPU). In addition, a plurality of processing units may be configured of one processor. As an example in which the plurality of processing units are configured of one processor, first, as typified by computers such as a client or a server, one processor is configured of a combination of one or more CPUs and software, and this processor functions as the plurality of processing units. Second, as typified by a system on chip (SoC) or the like, a processor that implements the functions of the entire system including the plurality of processing units by using one integrated circuit (IC) chip is used. As described above, the various processing units are configured using one or more of the various processors as a hardware structure.

Further, the hardware structure of these various processors is more specifically an electric circuit (circuitry) in a form in which circuit elements such as semiconductor elements are combined. The hardware structure of the storage unit is a storage device such as a hard disc drive (HDD) or a solid state drive (SSD).

In addition, in the above-described embodiment, the endoscope 12 to be combined with the ultrasonic treatment tool of the embodiment of the present invention is not specified, and the endoscope 12 need only comprise a forceps channel into which the treatment tool is inserted, for example, a bronchoscope, an upper gastrointestinal endoscope, or a lower gastrointestinal endoscope.

### Explanation of References

10: endoscope system
12: endoscope
12a: insertion part
12b: endoscope operating part
12c: bendable part
12d: distal end portion
12e: angle knob
14: light source device
15: processor device
16: display
17: user interface
18: ultrasonic treatment tool
19: ultrasonic driving part
20: ultrasonic treatment tool device
21: forceps port
22: forceps outlet
23: forceps channel
24: forceps valve
31: flexible sheath
32: operation wire
32A: connecting member
33: grip part
34: ultrasound oscillator unit
35: treatment tool operating part
36: operating part main body
36A: finger hook portion
36B: cylindrical portion
36C: connector portion
37: slider
41: gripping piece
41A: inner surface
41B: support shaft
42: link mechanism
42A: link plate
42B: connecting pin
42C: fitting pin
43: support member
43A: through-hole
43B: notch
51, 51a, 51b: ultrasound oscillator
52: backing material layer
53: acoustic matching layer
54a, 54b: piezoelectric material
56a, 56b: electrode
57a, 57b: electrode
58: air gap layer
61: signal transmitter
61a: frequency monitor
62: amplifier
63: impedance matching circuit
65: control unit
66: user interface
68: ultrasound oscillator
71: current probe
72: current value monitor
73: biomimetic material
S: structure
D1: first direction
D2: second direction
L: distance
V: ultrasonic wave

## Claims

1. An ultrasonic treatment tool device comprising:
a grip part that grips a structure in a subject by a pair of gripping pieces provided at a distal end portion of the grip part;
an ultrasound oscillator that is provided in at least one of a pair of the grip parts and emits an ultrasonic wave; and
a processor that drives the ultrasound oscillator such that the ultrasonic wave propagates in a first direction from one side toward the other side and propagates in a second direction opposite to the first direction in the structure, and that performs a frequency fluctuation mode for driving the ultrasound oscillator by changing a frequency of the ultrasound oscillator such that a current value flowing through the ultrasound oscillator is a maximal current value.

2. The ultrasonic treatment tool device according to claim 1,
wherein, in the frequency fluctuation mode, the frequency of the ultrasound oscillator is changed in a frequency range including a resonance frequency of the ultrasound oscillator.

3. The ultrasonic treatment tool device according to claim 1 or 2,
wherein the processor monitors the current value of an overlapping portion between a first range showing a waveform of the current value for the ultrasonic wave in the first direction and a second range showing a waveform of the current value for the ultrasonic wave in the second direction, and performs a control to maintain the frequency of the ultrasound oscillator at which the current value of the overlapping portion is the maximal current value.

4. The ultrasonic treatment tool device according to any one of claims 1 to 3,
wherein the structure includes a blood vessel.

5. The ultrasonic treatment tool device according to any one of claims 1 to 4,
wherein the ultrasonic treatment tool device is an endoscope treatment tool that is inserted into the subject through a forceps channel of an endoscope.

6. A method for driving an ultrasonic treatment tool device including a grip part that grips a structure in a subject by a pair of gripping pieces provided at a distal end portion of the grip part, and an ultrasound oscillator that is provided in at least one of a pair of the grip parts and emits an ultrasonic wave, the method comprising:
via a processor that drives the ultrasound oscillator such that the ultrasonic wave propagates in a first direction from one side toward the other side and propagates in a second direction opposite to the first direction in the structure,
a step of performing a frequency fluctuation mode for driving the ultrasound oscillator by changing a frequency of the ultrasound oscillator such that a current value flowing through the ultrasound oscillator is a maximal current value in a fixed frequency range with respect to the frequency of the ultrasound oscillator.
